# EUROPEAN PATENT APPLICATION

(11) **EP 2 754 710 A1**
(43) Date of publication of application: **16.07.2014**
(21) Application number: 12829423.8
(22) Date of filing: 06.09.2012
(51) Int. Cl.: C12N 1/12, A23K 1/16, A23L 1/30, A61K 31/734, A61P 1/12, A61P 3/06, A61P 3/10, A61P 9/10, A61P 9/12, B01J 20/24, B01J 20/30, C12P 7/44, C12R 1/89

(54) **METHOD FOR CULTIVATING SEAWEED AND METHOD FOR PRODUCING ALGINIC ACID-CONTAINING COMPOSITION**

(30) Priority: 09.09.2011 JP 2011197212
(71) Applicant: Kaneka Corporation, Osaka-shi, Osaka 530-8288 (JP)
(72) Inventor: KIZAKI, Noriyuki, Takasago-shi Hyogo 676-8688 (JP); FURUTA, Takeshi, Takasago-shi Hyogo 676-8688 (JP); MOURI, Taku, Takasago-shi Hyogo 676-8688 (JP); TAOKA, Naoaki, Takasago-shi Hyogo 676-8688 (JP)
(74) Representative: Vossius & Partner
(86) International application number: PCT/JP2012/072771
(87) International publication number: WO 2013/035797

(57) **Abstract**

The objective of the present invention is to provide a method for cultivating an alga having ability to produce alginic acid industrially and effectively and a method for producing an alginic acid-containing composition industrially advantageously. The method for cultivating an alga according to the present invention is characterized in comprising the step of cultivating the alga heterotrophically, wherein the alga belongs to the genus Parachlorella and has ability to produce alginic acid.

## Description

### TECHNICAL FIELD

The present invention relates to a method for cultivating an alga which belongs to the genus Parachlorella and which has ability to produce alginic acid. In addition, the present invention relates to a method for producing an alginic acid-containing composition industrially advantageously. Further, the present invention relates to a dried alga which is obtainable from the alginic acid-containing composition produced by the production method, and a reduction agent of sludge, a harmful substance adsorption agent, a feed, a food and a medical drug which contain the alginic acid-containing composition.

### BACKGROUND ART

Alginic acid is a polyuronide which is composed by binding two kinds of uronic acid, i.e. D-mannuronic acid and L-guluronic acid, to each other in various ratios, and is a high viscosity polysaccharide which is contained between cells and in cell wall of a brown alga and some red algae. Alginic acid and a salt thereof are widely utilized as a thickening agent and a gelling agent for a food, a medical drug, cosmetics, a dental material and the like, and attract attention as a functional food since alginic acid is a kind of dietary fiber. In particular, it has been reported that low-molecular-weight alginic acid, which is referred to as alginate oligomer, among alginic acids exhibits various bioactivities (Non-patent Document 1).

Alginic acid and a salt thereof have been industrially produced by extracting and purifying from a brown alga such as laminaria and brown seaweed. However, in the case that a brown alga is used as raw material for producing alginic acid, there is a problem with stable supply of the raw material.

In addition, as a method for producing aliginate oligomer, the following methods are known:
a method in which high-molecular-weight alginic acid obtained from a brown alga is used as a raw material and the molecular weight is reduced by treating the alginic acid with an enzyme or a bacterium which has ability to decompose alginic acid (Patent Documents 1 and 2); and
a method in which the molecular weight of the high-molecular-weight alginic acid is reduced by heat treatment under pressure (Patent Documents 3 and 4).

However, the former method has a disadvantage for industrial production, since the method requires a large cost due to the use of an enzyme or a bacterium and it is necessary to react for a long time with using a low-concentration aqueous solution. The latter pressure heat treatment method has a problem in facilities, since the method requires an expensive pressure vessel.

Recently, a novel alga which belongs to the genus Parachlorella and which has ability to produce alginic acid, particularly alginic acid having a relatively low molecule, was found. It was reported in Patent Document 5 that the alga is cultivated and alginic acid is obtained from the culture broth. In addition, a method for reducing sludge by using the dried alga product containing the obtained alginic acid was reported (Patent Document 6). In the above Patent Documents, it is described that the novel alga requires light. In fact, in a method described in the Patent Documents for cultivating the above-described alga, photoautotrophic cultivation was carried out by using the photosynthetic ability of the alga.

In general, an alga can be autotrophically cultivated by using the photosynthetic ability. Photoautotrophic cultivation has advantage that natural light such as sunlight can be utilized and it is not necessary that carbon source and energy source are added to a culture medium. However, photoautotrophic cultivation has problems that irradiation light quantity is changed due to weather and season. In addition, with respect to facilities, a culture broth tends to become contaminated by microorganism and the like. As a method to solve the above problems, there is a method for cultivating by radiating artificial light such as electric light in a completely closed system. However, such a method requires expensive facilities and much electric energy.

### PRIOR ART

### PATENT DOCUMENT

Patent Document 1: JP H2-303468 A
Patent Document 2: JP H5-15387 A
Patent Document 3: JP H6-7093 A
Patent Document 4: JP 2006-320320 A
Patent Document 5: WO 2010-024367
Patent Document 6: JP 4573187 B

### NON-PATENT DOCUMENT

Non-patent Document 1: Japanese Journal of Food Chemistry and Safety, 17, pp. 27-35 (2010)

### DISCLOSURE OF THE INVENTION

### PROBLEMS TO BE SOLVED BY THE INVENTION

Under the above-described circumstance, the objective of the present invention is to provide a method for cultivating an alga having ability to produce alginic acid industrially and effectively. In addition, the objective of the present invention is to provide a method for producing an alginic acid-containing composition industrially advantageously.

### MEANS FOR SOLVING THE PROBLEMS

As a method for cultivating an alga which belongs to the genus Parachlorella and which has ability to produce alginic acid, heterotrophic cultivation for which a light is necessary has been known only. On the other hand, the inventors of the present invention surprisingly found that the alga can be heterotrophically cultivated with high efficiency. In addition, the inventors found that when the alga is heterotrophically cultivated, more amount of alginic acid can be accumulated in the cultured product in comparison with the case of autotrophic cultivation. As a result, the inventors completed the present invention.

The present invention is described as follows.
[1] A method for cultivating an alga,
   comprising the step of cultivating the alga heterotrophically,
   wherein the alga belongs to the genus Parachlorella and has ability to produce alginic acid.
[2] The method according to the above [1], wherein the alga belonging to the genus Parachlorella is Parachlorella sp. binos FERM BP-10969.
[3] The method according to the above [1] or [2], wherein the alga is cultivated under dark condition of less than 700 Lux on average illuminance.
[4] A method according to any one of the above [1] to [3], wherein the alga is cultivated in a liquid culture medium, and a total amount of an organic compound to be used in the liquid culture medium is adjusted to not less than 2.5 g in terms of carbon atom weight relative to 1 L of the liquid culture medium at the start of the cultivation.
[5] A method for producing an alginic acid-containing composition, comprising the step of obtaining the alginic acid-containing composition from a culture obtained by the method according to any one of the above [1] to [4].
[6] A dried alga, wherein
   the dried alga is a dried product of the alginic acid-containing composition obtained by the method according to the above [5], and
   a content of alginic acid per 1 kg is not less than 40 g.
[7] A reduction agent of sludge, comprising the alginic acid-containing composition obtained by the method according to the above [5].
[8] A harmful substance adsorption agent, comprising the alginic acid-containing composition obtained by the method according to the above [5].
[9] A feed, comprising the alginic acid-containing composition obtained by the method according to the above [5].
[10] A food, comprising the alginic acid-containing composition obtained by the method according to the above [5].
[11] A medical drug, comprising the alginic acid-containing composition obtained by the method according to the above [5].

### EFFECT OF THE INVENTION

According to the present invention, an alga which has ability to produce alginic acid can be effectively propagated using a general microorganism cultivation tank without supplying a light during cultivation. In addition, according to the present invention, a method for industrially producing an alginic acid-containing composition with good efficiency can be provided. The alginic acid-containing composition obtained by the present invention method contains much amount of alginic acid and is useful as a raw material for a reduction agent of sludge, a harmful substance adsorption agent, a feed, a food and a medical drug.

### MODE FOR CARRYING OUT THE INVENTION

The method for cultivating an alga is characterized in comprising the step of heterotrophically cultivating an alga which has ability to produce alginic acid.

The alga used in the present invention has ability to produce alginic acid and belongs to the genus Parachlorella.

The alga belonging to the genus Parachlorella was once classified into the genus Chlorella in the class Trebouxiophyceae. However, the genus Parachlorella was acknowledged as a genus which is independent and different from the genus Chlorella, since the alga forms a group which is different from other algae belonging to the genus Chlorella in the class Trebouxiophyceae by molecular phylogenic analysis of 18S rDNA and 16S rDNA.

The alga belonging to the genus Parachlorella is exemplified by Parachlorella sp. binos, Parachlorella kessleri and Parachlorella beijerinckii. However, the alga used for the present invention is not limited to the above examples. With respect to an alga of which biological species is not identified, such an alga may be used in the present invention as long as the alga belongs to the genus Parachlorella or an allied genus thereof and has ability to produce alginic acid.

In the present invention, an alga which belongs to an allied genus of the genus Parachlorella means an alga which belongs to the genera adjacent to the genus Parachlorella in the molecular phylogenetic tree obtained by molecular phylogenic analysis of 18S rDNA and 16S rDNA. In other words, an alga which has ability to produce alginic acid and which belongs to the genus Closteriopsis, the genus Dicloster or the genus Marinichlorella can be used in the present invention.

Even if it has not been known that a certain alga can be heterotrophically cultivated, such an alga may be possibly used in the present invention. In other words, even though a certain alga has a photosynthetic capacity and has been autotrophically cultivated, the alga which can be heterotrophically cultivated and has ability to produce alginic acid can be used in the present invention.

In general, the alga belonging to the genus Parachlorella can be obtained by separating a colony of an alga by subcultivation from a sample of fresh water obtained out of doors using a general culture medium and finally identifying a genus and a species by molecular phylogenic analysis.

The alga used in the present invention has ability to produce alginic acid. The produced alginic acid may be exclusively secreted from a cell of the alga belonging to the genus Parachlorella or may be accumulated in the cell. When alginic acid is detected in a culture broth of the alga belonging to the genus Parachlorella or homogenate of the culture, it is judged that the alga belonging to the genus Parachlorella has ability to produce alginic acid.

Alginic acid is a polymer which is composed by randomly polymerizing D-mannuronic acid and L-guluronic acid. In general, the polymerization degree of alginic acid, which corresponds to the number of monosaccharide unit which composes alginic acid, is not less than about 80 and not more than about 1100, and the molecular weight is not less than about 15, 000 and not more than about 200, 000. However, in the present invention, an alginate oligomer having smaller molecular weight is included in the definition of alginic acid. The polymerization degree of an alginate oligomer is not less than about 3 and not more than about 10, and the molecular weight is not less than about 500 and not more than about 2000.

In the present invention, not only free alginic acid but also an alginate salt is included in the definition of alginic acid. Such an alginate salt is exemplified by a sodium salt, a calcium salt, a magnesium salt, a potassium salt and an ammonium salt. For example, the alginic acid produced by the cultivation method according to the present invention can be quantitated by naphthoresorcinol color reaction method, which is described in Journal of the Food Hygienic Society of Japan, vol.39, no.5, p.297 (1998). In the present invention, a quantitative value of alginic acid is demonstrated as an amount calculated in terms of sodium alginate.

As a preferred specific example of the alga used in the present invention, Parachlorella sp. binos FERM BP-10969 is exemplified. The strain "FERM BP-10969" was deposited at a depositary institution as follows.
(i) Name and Address of depositary institution
   Name: National Institute of Advanced Industrial Science and Technology - International Patent Organism Depositary
   Address: Chuo-Dai 6, 1-1-1, Tsukuba-shi, Ibaragi, Japan
(ii) Date of receipt: February 28, 2008
(iii) Accession number: FERM P-21513
(iv) International accession number: BP-10969
(v) Date of conversion from deposit under the national act to that under the Budapest Treaty: May 23, 2008

In the present invention, as the alga which belongs to the genus Parachlorella and which has ability to produce alginic acid, a mutant strain or a hybridized strain of the above-described Parachlorella sp. binos FERM BP-10969 are preferably used in addition to Parachlorella sp. binos FERM BP-10969.

A mutant strain which is used in the present invention and which is obtained from FERM BP-10969 means a FERM BP-10969 mutant having some mutation and having ability to produce alginic acid. A hybridized strain which is used in the present invention and which is obtained from FERM BP-10969 means a hybridized strain which has ability to produce alginic acid and which is obtained by crossbreeding FERM BP-10969 with a strain which is taxonomically classified in the same species to FERM BP-10969 but which is relatively-distinctly related to FERM BP-10969.

The strain FERM BP-10969 contains chlorophyll in a dry weight amount of more than two times than that of a general alga belonging to the genus Chlorella and has very high photosynthetic ability. Therefore, the strain FERM BP-10969 has been autotrophically cultivated naturally. On the other hand, the inventors of the present invention found that alginic acid can be produced very efficiently by daringly cultivating FERM BP-10969 heterotrophically.

In the present invention, the term "heterotrophical cultivation" means cultivation under dark condition using a culture medium containing an organic compound as energy source and/or carbon source. However, when a culture medium contains enough amount of organic compound to be utilized as energy source and/or carbon source so that photosynthesis is not necessary, the alga may be cultivated under light condition.

In the present invention, the term "under dark condition" means a condition that enough light to proliferate alga cell is not applied to a culture broth. Specifically, the condition means a condition that an average illumination intensity is less than 700 Lux, preferably less than 300 Lux, more preferably less than 100 Lux, even more preferably less than 10 Lux, and further preferably less than 1 Lux. It is possible to carry out cultivation with satisfying the above-described average illumination intensity condition by using a shading means which is generally used or using a fermenter which is generally used for heterotrophical cultivation. In general, an illumination intensity is changed depending on a distance from light source, a density of alga cell and the like, since light is attenuated due to the presence of a culture medium and increased alga cell. Therefore, for example, an average illumination intensity in a culture broth is obtained by measuring illumination intensities at multiple positions in a culture broth which is placed in a similar condition to that of cultivation and calculating an average value of the measured values. It is preferred that an illumination intensities are measured at not less than two points and not more than 12 points in which at least the surface and the deepest portion of a culture broth are included.

In the present invention, an organic compound which is used as energy source and/or carbon source when the alga having ability to produce alginic acid is cultivated heterotrophically is not particularly limited as long as the alga can assimilate the organic compound. Such an organic compound is exemplified by a carbohydrate such as glucose, galactose, sucrose, xylose, maltose, cellobiose, trehalose, lactose, mannitol, various oligosaccharides and starch; an alcohol such as methanol, ethanol, glycerol, ethylene glycol and propylene glycol; an organic acid such as citric acid, succinic acid, malic acid, fumaric acid, butyric acid, propionic acid and acetic acid; an amino acid such as glutamic acid, glutamine, aspartic acid, asparagine, glycine, proline, lysine, alanine, leucine, valine, isoleucine, phenylalanine, tyrosine, cysteine, histidine, serine, tryptophan, threonine and methionine; an oil and fat, such as a plant oil and fat, and a fatty acid. One of the above-described organic compounds may be used alone, or the organic acids may be optionally combined to be used. It is not always necessary to purify the above-described organic compound to be used. For example, unpurified compound such as molasses, milk whey and starch hydrolyzate can be used as energy source and/or carbon source.

In the present invention, a total amount of organic compound which is added in a culture medium as energy source and/or carbon source when the alga having ability to produce alginic acid is cultivated heterotrophically is not particularly limited as long as heterotrophical cultivation can be carried out. The total amount relative to 1 L of a culture medium at the start of cultivation is exemplified by not less than 2.5 g, preferably not less than 3 g, more preferably not less than 5 g, even more preferably not less than 8 g, further preferably not less than 10 g, and particularly preferably not less than 50 g. When a culture medium is further added during cultivation or feeding cultivation is carried out, the above total amount means the sum of the amounts of the culture media.

In the present invention, a culture medium used when the alga having ability to produce alginic acid is cultivated heterotrophically is not particularly limited as long as the alga having ability to produce alginic acid can be proliferated heterotrophically. For example, such a culture medium may contain nitrogen source, phosphorus source, a mineral and others in addition to the above-described organic compound used as energy source and/or carbon source. The culture medium may further contain a vitamin, an antifoaming agent, a pH adjuster and others. The above-described nitrogen source is exemplified by ammonia and a salt thereof, urea, nitric acid and a salt thereof, and an amino acid. The above-described phosphorus source is exemplified by phosphoric acid and a salt thereof and polyphosphoric acid. The above-described mineral is exemplified by boric acid in addition to a salt of a metal such as manganese, zinc, copper, sodium, potassium, iron, calcium and molybdenum. The above-described vitamin is exemplified by vitamin A, B1, B2, B6, B12, C, D, E, K, niacin, pantothenic acid, folic acid, biotin, and a precursor and a derivative thereof. It is not always necessary to purify such nitrogen source, phosphorus source, mineral and vitamin and other culture medium component to be used. For example, a natural compound such as yeast extract and peptone may be used. The above compounds may be optionally combined to be used.

In the present invention, the alga having ability to produce alginic acid can be heterotrophically cultivated by adding the alga having ability to produce alginic acid into a container which contains a culture medium containing the above-described organic compound as energy source and/or carbon source, nitrogen source, mineral, vitamin and other component and incubating the alga preferably with aeration and/or with stirring the culture broth. A cultivation temperature is preferably adjusted to a range of not less than 10°C and not more than 50°C, more preferably a range of not less than 20°C and not more than 40°C, and even more preferably a range of not less than 25°C and not more than 30°C. When a cultivation temperature is less than 10°C or more than 50°C, a growth rate of the alga having ability to produce alginic acid may be markedly decreased. Therefore, such a condition is not suitable for industrial cultivation. A pH value during cultivation is preferably adjusted to a range of not less than 3.0 and not more than 10.0, more preferably a range of not less than 4.0 and not more than 9.0, and even more preferably a range of not less than 5.0 and not more than 8.0. When a pH value during cultivation is less than 3.0 or more than 10.0, a growth rate of the alga having ability to produce alginic acid may be markedly decreased. Therefore, such a condition is not suitable for industrial cultivation.

In the present invention, a component of the above-described culture medium used when the alga having ability to produce alginic acid is heterotrophically cultivated may be added in a lump before beginning cultivation or in a divided manner and/or in a continuous manner during cultivation. However, it is preferred that an organic compound used as energy source and/or carbon source is added in a divided manner and/or in a continuous manner, since large amount of the organic compound is necessary to increase a density of the alga cell in a culture broth. In such a case, it is preferred that a concentration of an organic compound used as energy source and/or carbon source in a culture medium is maintained to be not more than 32 g/L, more preferably not more than 16 g/L, even more preferably not more than 8 g/L, and further preferably not more than 4 g/L, in terms of a weight of carbon atom contained in the organic compound.

The culture medium for the alga having ability to produce alginic acid obtained by the present invention method may be changed to a culture medium which does not contain an organic compound used as energy source and/or carbon source for subsequent cultivation as necessary. In such a case, the cultivation may be carried out in either of dark condition or light condition. In particular, a culture product obtained by such a two-stage cultivation can be preferably used as a raw material of a reduction agent of sludge or a harmful substance adsorption agent.

In order to ensure a sufficient amount of the alga used in the present invention, the alga may be pre-cultivated. Such a precultivation may be carried out in either of dark condition or light condition.

According to the present invention method for cultivation, a yield of the alga per a culture broth can be remarkably increased and a yield of alginic acid can be also increased in comparison with a conventional autotrophic cultivation. A culture broth which is obtained by cultivating the alga having ability to produce alginic acid according to the present invention may preferably contain not less than 2 g of the dried alga per 1 L of the culture broth at the end of the cultivation. The amount of the dried alga is more preferably not less than 3 g, even more preferably not less than 5 g, further preferably not less than 10 g, and particularly preferably not less than 50 g.

For example, a concentration of the dried alga in a culture broth can be measured by the following method. A culture broth was taken in a certain amount. The culture broth is centrifuged and the supernatant is removed. The obtained alga is dispersed again in water in an amount equal to that of the culture broth. The dispersion is centrifuged again and the supernatant used for washing the alga is removed. The washed alga is dried until the weight thereof is not further decreased, and a weight of the dried alga is measured. The measured weight of the dried alga is divided by the amount of the taken culture broth to calculate a concentration of the dried alga.

In addition, surprisingly, according to the present invention method for cultivation, productivity of alginic acid per alga amount can be improved. According to the present invention method for cultivation, productivity in terms of sodium alginate per 1 kg of the dried alga is usually not less than 40 g, preferably not less than 50 g, more preferably not less than 100 g, and particularly preferably not less than 150 g.

According to the present invention method for cultivation, productivity of alginic acid per an amount of a culture broth is remarkably improved by both of the above-described improvement of the alga yield per a culture broth and improvement of alginic acid productivity per the alga body in comparison with a conventional autotrophic cultivation method. According to the present invention method for cultivation, alginic acid in terms of sodium alginate per 1 L of a culture broth at the end of cultivation can be accumulated preferably in an amount of not less than 10 mg, more preferably not less than 100 mg, even more preferably not less than 500 mg, further preferably not less than 1000 mg, and particularly preferably not less than 5000 mg.

Also, the present invention relates to a method for producing an alginic acid-containing composition, characterized in comprising the step of obtaining the alginic acid-containing composition from a culture obtained by the above-described present invention method for cultivation. In other words, a method for producing an alginic acid-containing composition by heterotrophically cultivating an alga which belongs to the genus Parachlorella and which has ability to produce alginic acid in order to obtain a culture product and using the obtained culture product is included in the range of the present invention.

The alginic acid-containing composition of the present invention is not particularly limited as long as the composition contains alginic acid and/or a salt thereof. The composition may contain a component derived from the alga or the crushed alga body in addition to alginic acid or may contain high purity alginic acid by removing the alga body and the like. In other words, the alginic acid-containing composition obtained by the production method of the present invention is exemplified by a culture broth itself obtained by cultivating the alga having ability to produce alginic acid, the alga body obtained from the culture broth, a concentrated culture broth of which alga concentration in the culture broth is increased, and/or a culture supernatant obtained by removing the alga body from the culture broth. In addition, the culture broth, alga body, concentrated culture broth and/or culture supernatant may be subjected to a treatment such as drying and purifying in order to increase a concentration of alginic acid and/or a salt thereof as necessary. Such a treated product is also included in the range of the alginic acid-containing composition obtained by the production method of the present invention. When the alginic acid-containing composition is used as the reduction agent of sludge or the harmful substance adsorption agent described later, it is preferred that the alginic acid-containing composition contains the alga body or the crushed alga body in addition to alginic acid.

In the present invention, a method for obtaining alginic acid-containing composition from a culture obtained by heterotrophically cultivating the alga having an ability to produce alginic acid is not particularly limited. For example, a culture supernatant, concentrated culture broth and/or alga body which contain alginic acid can be obtained by carrying out the above-described cultivation method of the present invention in order to obtain a culture broth containing alginic acid and subjecting the culture broth to a treatment such as centrifugation and filtration. The culture broth, culture supernatant, concentrated culture broth and/or alga body may be dried by a drying method such as freeze-drying, spray-drying, drum-drying and reduced pressure-drying. In addition, the alginic acid-containing composition having higher concentration can be produced from the culture broth, culture supernatant, concentrated culture broth, alga body and/or dried product thereof by using one conventional method or combining conventional methods to be used as necessary. A method for obtaining the alginic acid-containing composition having higher concentration is exemplified by a method in which an acid such as sulfuric acid is added to a culture supernatant to insolubilize alginic acid and the insolubilized alginic acid is obtained by a treatment such as centrifugation and filtration, a method in which a metal salt such as calcium chloride is added to a culture supernatant for gelling alginic acid and the gelled alginic acid is obtained by a treatment such as centrifugation and filtration, and a method in which a water-soluble organic solvent such as ethanol, acetone and isopropanol is added to a culture supernatant to insolubilize alginic acid and the insolubilized alginic acid is obtained by a treatment such as centrifugation and filtration. In addition, for example, an alginate salt such as sodium alginate can be purified from the alga body by subjecting the alga body to washing procedure and/or extracting procedure using an alkaline aqueous solution such as sodium carbonate aqueous solution and carrying out a treatment such as centrifugation and filtration to remove an insoluble component. For example, the alginic acid-containing composition having much higher concentration can be produced by subjecting the obtained alginate salt aqueous solution to similar treatment to the case of the above-described culture supernatant.

Also, the present invention relates to a reduction agent of sludge or a harmful substance adsorption agent which comprise the alginic acid-containing composition obtained by the above-described production method of the present invention.

In the present invention, a reduction agent of sludge means a composition which can reduce an amount of a finally-remaining waste sludge by adding the composition to activated sludge used in a treatment of organic wastewater with activated sludge method.

A condition to use the reduction agent of sludge according to the present invention may be optionally determined. For example, the reduction agent of sludge according to the present invention in an amount of not less than about 20 g and not more than about 200 g in terms of dried condition may be added to 1 ton of sludge. After the addition, it is preferred that the mixture is stirred or bubbled in order to ensure sufficient dissolved oxygen amount. A treatment temperature may be ordinary temperature. A treatment time may be adjusted within a range of not less than 10 hours and not more than 10 days depending on an amount of sludge to be treated.

A harmful substance adsorption agent means a composition which is used for removing a part or all of harmful substance in pollutant by adsorbing harmful substance in pollutant when the harmful substance adsorption agent is brought into contact with the pollutant and then separating the harmful substance adsorption agent from the pollutant. A harmful substance is exemplified by a radioactive element such as cesium 137, cesium 134, strontium 89, strontium 90, strontium 91, iodine 131 and iodine 133; a heavy metal such as lead, copper, cadmium and arsenic; and a compound containing the above substance; however, a harmful substance is not limited to the above examples. A condition to use the harmful substance adsorption agent of the present invention may be optionally determined similarly to the condition to use the above-described reduction agent.

In the present invention, a method for producing the reduction agent of sludge or harmful substance adsorption agent which contains the alginic acid-containing composition is not particularly limited. For example, a cultured product obtained by the cultivation method of the present invention is further cultivated using a culture medium which does not contain an organic compound as energy source and/or carbon source if necessary, and then the cultured product is concentrated or dried to obtain the alginic acid-containing composition which can be obtained by the production method of the present invention. The thus obtained alginic acid-containing composition may be directly used or may be optionally processed to be used.

Also, the present invention relates to a feed, a food or a medical drug which comprises the alginic acid-containing composition obtained by the above-described production method of the present invention.

In the present invention, a food means a substance to be ingested by a human to take nutrient or for taste, and a food additive and a supplement such as a dietary supplement and a food for specified health use are included in a range of the food in addition to a general food which is usually eaten.

In the present invention, a feed means a substance to be ingested by an organism other than a human mainly to take nutrient. A feed is exemplified by a feed, a feed additive and a supplement for an aquatic organism such as fish, crustacea and shellfish; domestic poultry such as chicken, quail and duck; livestock such as cattle, a swine and a sheep; and a pet such as a dog and a cat. However, a feed is not limited to the above examples.

In the present invention, a medical drug means a substance which is used for preventing or treating a disease and a symptom of a human and an other animal.

The alginic acid-containing composition obtained by the production method of the present invention can be used for the above-described feed, food and medical drug by optionally processing into a powder, a tablet, a capsule and others or by mixing with a food raw material or a feed raw material. The feed, food or medical drug comprising the alginic acid-containing composition of the present invention can be used for adding thickness to a food and a formulation and used as a composition containing dietary fiber due to physical properties of alginic acid, and additionally can be used for preventing and treating high blood pressure, decreasing blood cholesterol, suppressing blood sugar value, curing constipation, and preventing and treating arteriosclerosis. In addition, the alginic acid-containing composition can be used as an agent to prevent radioactive strontium such as strontium 89, strontium 90 and strontium 91 from remaining in a body due to a function of alginic acid to discharge strontium from a body.

The present application claims the benefit of the priority date of Japanese patent application No. 2011-197212 filed on September 9, 2011, and all of the contents of the Japanese patent application No. 2011-197212 filed on September 9, 2011 are incorporated by reference.

### EXAMPLES

Hereinafter, the present invention is described in more detail with Examples. In the following Examples, a content of dried alga in a culture broth and a concentration of alginic acid in a culture broth were measured as follows unless specified otherwise.

### Content of dried alga in culture broth

A certain amount of culture broth was centrifuged at 3000 × G and the supernatant was removed. Then, the precipitated alga was dispersed again in 5 mL of water, and the dispersion was similarly centrifuged and the supernatant was removed. The water-washed alga was dried at 110°C for 8 hours until the weight was not further decreased, and then the dried alga was weighed. From the result, a weight of dried alga contained in 1 L of a culture broth was calculated.

### Concentration of alginic acid in culture broth

A culture broth was properly diluted using water, and 2 mL of a copper - hydrochloric acid reagent and 1 mL of a naphthoresorcinol reagent were added to 1 mL of the diluted culture broth. The mixture was heated in boiled water for 65 minutes. The above copper - hydrochloric acid reagent was prepared by adding 1 mL of 2.5% copper sulfate aqueous solution and 9 mL of water to 40 mL of concentrated hydrochloric acid. The above naphthoresorcinol reagent was prepared by dissolving 100 mg of 1,3-dihydroxynaphthalene in 25 mL of water. The above mixture was cooled in iced water, and 4 mL of butyl acetate was added thereto. The mixture was stirred and then centrifuged. The butyl acetate layer was filtered through a filter having a pore diameter of 0.45 µm (COSMONICE Filter S, manufactured by NACALAI TESQUE, INC.), and alginic acid was determined by the colorimetry at 566 nm. The concentration of alginic acid in the sample was calculated in terms of sodium alginate by using a calibration curve. The calibration curve was prepared by using sodium alginate having a viscosity of 300 to 400 cP (manufactured by Wako Pure Chemical Industries, Ltd.) as a standard substance.

### Example 1

Into a flask for shaking cultivation, 100 mL of a culture medium of which pH value was 6.5 was added. The composition of the culture medium was 20 g/L of glucose, 1 g/L of potassium dihydrogenphosphate, 1 g/L of magnesium sulfate heptahydrate, 0.01 g/L of calcium chloride, 2 g/L of urea, 5 mg/L of ferrous sulfate heptahydrate and 1 mL/L of Arnon's A5 solution. The composition of the Arnon's A5 solution was 2.86 g/L of boric acid, 1.81 g/L of manganese chloride tetrahydrate, 0.08 g/L of copper sulfate tetrahydrate, 0.22 g/L of zinc sulfate heptahydrate and 0.171 g/L of ammonium molybdate tetrahydrate. After the flask was sterilized, Parachlorella sp. binos FERM BP-10969 was inoculated thereto. After the flask was shielded using an aluminum foil, the alga was cultivated with shaking the flask at 30°C for 72 hours. After the cultivation, the content of the dried alga in the obtained culture broth was measured to be 9.0 g/L. In addition, the concentration of alginic acid in the obtained culture broth was measured to be 0.52 g/L in terms of sodium alginate. From the results, the production volume of alginic acid per 1 kg of the dried alga was 58 g.

### Example 2

Into a flask for shaking cultivation, 100 mL of the culture medium which had the same composition described in Example 1 was added. After the flask sterilized, Parachlorella sp. binos FERM BP-10969 was inoculated thereto. After the flask was shielded using an aluminum foil, the alga was cultivated in the dark with shaking the flask at 30°C for 72 hours as precultivation. Then, into a 5 L jar fermenter, 2000 mL of a culture medium was added. The composition of the culture medium was 10 g/L of glucose, 1.5 g/L of potassium dihydrogenphosphate, 1.5 g/L of magnesium sulfate heptahydrate, 20 mg/L of calcium chloride, 3.5 g/L of urea, 10 mg/L of ferrous sulfate heptahydrate, 0.1 g/L of citric acid, 1 mL/L of Arnon's A5 solution of which composition was described in Example 1 and 0.1 g/L of ADEKA NOL LG109 manufactured by ADEKA Corporation. After the jar fermenter was sterilized, 200 mL of the culture broth obtained in the above-described precultivation was added thereto. After the jar fermenter was shielded using an aluminum foil, the alga was cultivated in the condition that inner temperature was 30°C, aeration rate was 2 L/min, agitation speed was 450 rpm and pH was 6 to 7 for 143 hours. The lower limit of pH was adjusted using 30 w/w% sodium hydroxide aqueous solution and the upper limit of pH was adjusted using 15 w/w% of aqueous sulfuric acid. After 23 hours from the beginning of cultivation, it was started to feed a sterilized fed-batch culture medium to the culture broth. The addition rate of the fed-batch culture medium was properly adjusted so that the concentration of glucose in the culture broth did not become more than 20 g/L. The amount of the fed-batch culture medium which was added by the end of the cultivation was totally 560 mL. The composition of the fed-batch culture medium was 570 g/L of glucose, 20 g/L of potassium dihydrogenphosphate, 12 g/L of magnesium sulfate heptahydrate, 1.25 g/L of calcium chloride, 42 g/L of urea, 700 mg/L of ferrous sulfate heptahydrate, 10 mL/L of Arnon's A5 solution and 4.5 g/L of citric acid.

After the cultivation, the content of the dried alga in the obtained culture broth was measured to be 54.3 g/L. In addition, the concentration of alginic acid in the obtained culture broth was measured to be 3.4 g/L in terms of sodium alginate. From the results, the production volume of alginic acid per 1 kg of the dried alga was 63 g.

### Example 3

Into a flask for shaking cultivation, 100 mL of a culture medium was added. The composition of the culture medium was 111 mM (20 g/L) of glucose, 50 mM of potassium nitrate, 9.2 mM of potassium dihydrogenphosphate, 0.57 mM of dipotassium hydrogenphosphate, 10 mM of magnesium sulfate, 31 mM of sodium chloride, 0.14 mM of EDTA, 0.11 mM of ferrous sulfate and 1 mL/L of Arnon's A5 solution of which composition was described in Example 1. After the flask was sterilized, Parachlorella sp. binos FERM BP-10969 was inoculated thereto. After the flask was shielded using an aluminum foil, the alga was cultivated with shaking the flask at 30°C for 72 hours. After the culture broth was centrifuged and the supernatant was removed, the alga was dispersed again in 100 mL of an inorganic salt culture medium. The composition of the inorganic salt medium was 2.5 mM of potassium nitrate, 1.3 mM of potassium dihydrogenphosphate, 0. 3 mM of magnesium sulfate, 0. 43 mM of sodium chloride, 0.23 mM of calcium chloride, 1.7 mM of ammonium dihydrogenphosphate, 0.047 mM of ferrous sulfate and 2 mL/L of Arnon's A5 solution. The alga was further cultivated with shaking the flask at 30°C for 16 hours. After the cultivation, the content of the dried alga in the obtained culture broth was measured to be 3.5 g/L. In addition, the concentration of alginic acid in the obtained culture broth was measured to be 0.14 g/L in terms of sodium alginate. From the results, the production volume of alginic acid per 1 kg of the dried alga was 40 g.

### Comparative Example 1: Autotrophic culture

Into a flask, 1000 mL of a culture medium of which pH was 6.5 was added. The composition of the culture medium was 2.5 g/L of potassium nitrate, 17.5 g/L of potassium dihydrogenphosphate, 7.5 g/L of magnesium sulfate heptahydrate, 2.5 g/L of calcium chloride, 2.5 g/L of sodium chloride, 20 g/L of ammonium dihydrogenphosphate, 6.5 mg/L of ferric chloride and 2 mL/L of Arnon's A5 solution of which composition was described in Example 1. After the flask was sterilized, Parachlorella sp. binos FERM BP-10969 was inoculated thereto. The alga was cultivated with stirring at 25°C under illumination of about 7000 Lux for 9 days. After the cultivation, the content of the dried alga in the obtained culture broth was measured to be 67 mg/L. In addition, the concentration of alginic acid in the obtained culture broth was measured to be 2.3 mg/L in terms of sodium alginate conversion. From the results, the production volume of alginic acid per 1 kg of the dried alga was 34 g.

### Example 4

Into a 2L flask for shaking cultivation, 400 mL of a culture medium of which pH was 6.5 was added. The composition of the culture medium was 20 g/L of glucose, 1 g/L of potassium dihydrogenphosphate, 1 g/L of magnesium sulfate heptahydrate, 0.01 g/L of calcium chloride, 2 g/L of urea, 5 mg/L of ferrous sulfate heptahydrate and 1 mL/L of Arnon's A5 solution. The composition of the Arnon's A5 solution was 2.86 g/L of boric acid, 1.81 g/L of manganese chloride tetrahydrate, 0.08 g/L of copper sulfate tetrahydrate, 0.22 g/L of zinc sulfate heptahydrate and 0.171 g/L of ammonium molybdate tetrahydrate. After the flask was sterilized, Parachlorella sp. binos FERM BP-10969 was inoculated thereto. After the flask was shielded using an aluminum foil, the alga was cultivated with shaking the flask at 30°C for 48 hours to obtain a preculture broth.

Into a 30 L jar fermenter, 10 L of a culture medium of which pH was 6.0 was added. The composition of the culture medium was 3 g/L of yeast extract, 11.5 g/L of potassium dihydrogenphosphate, 7.5 g/L of magnesium sulfate heptahydrate, 0.65 g/L of calcium chloride, 2.4 g/L of citric acid, 0.36 g/L of ferrous sulfate heptahydrate and 1 mL/L of Arnon's A5 solution. The composition of the Arnon's A5 solution was 2.86 g/L of boric acid, 1.81 g/L of manganese chloride tetrahydrate, 0.08 g/L of copper sulfate tetrahydrate, 0.22 g/L of zinc sulfate heptahydrate and 0.171 g/L of ammonium molybdate tetrahydrate. The culture medium was steamed to be sterilized in the 30 L jar fermenter. The culture broth of the above-described precultivation was added to the jar fermenter, and the alga was cultivated in a condition of 18 L/min of aeration rate, 30°C and pH 6.0 for 120 hours. During cultivation, 55 wt% of sterilized glucose aqueous solution was continuously in a total amount of 5.4 kg, and an agitation speed was adjusted so that a dissolved oxygen concentration of the culture broth become 0.1 ppm or more. In order to adjust a pH value of the culture broth, 25 wt% of aqueous ammonia was used.

After the cultivation, the content of the dried alga in the obtained culture broth was measured to be 93.0 g/L. In addition, the concentration of alginic acid in the obtained culture broth was measured to be 15.8 g/L in terms of sodium alginate. From the results, the production volume of alginic acid per 1 kg of the dried alga was 170 g.

### Example 5

An alginic acid-containing composition was obtained by freeze-drying, spray-drying or drum-drying the culture broth of Parachlorella sp. binos FERM BP-10969 obtained in Example 2. Freeze-drying was carried out using Freeze Dryer FD-1 manufactured by Tokyo Rikakikai Co., LTD., spray-drying was carried out using Mini Spray Dryer B-290 manufactured by BÜCHI Labortechnik AG, and drum-drying was carried out using Double drum type drum dryer of which drum size was ϕ400 × L500 mm and which was manufactured by KATSURAGI IND. CO., LTD. Contents of alginic acid in the dried compositions obtained in the above were measured; as a result, the content in the freeze dried composition was 16.4 wt%, the content in the spray-dried composition was 15.7 wt% and the content in the drum-dried composition was 15.2 wt%.

### Comparative Example 2: Autotrophic culture

Into a 1 L conical flask, 500 mL of a culture medium of which pH was 7.0 was added. The composition of the culture medium was 175 mg/L of potassium dihydrogenphosphate, 200 mg/L of ammonium dihydrogenphosphate, 75 mg/L of magnesium sulfate heptahydrate, 25 mg/L of calcium chloride, 25 mg/L of sodium chloride, 250 mg/L of potassium nitrate, 5 mg/L of ferric sulfate heptahydrate and 1 mL/L of Arnon's A5 solution. The composition of the Arnon's A5 solution was 2.86 g/L of boric acid, 1.81 g/L of manganese chloride tetrahydrate, 0.08 g/L of copper sulfate tetrahydrate, 0.22 g/L of zinc sulfate heptahydrate and 0.171 g/L of ammonium molybdate tetrahydrate. To the conical flask, Parachlorella sp. binos FERM BP-10969 was inoculated thereto. Then, the alga was cultivated with bubbling by carbon dioxide gas at 25°C under illumination of about 7000 Lux for 10 days. After the cultivation, the content of the dried alga in the obtained culture broth was measured to be 850 mg/L. In addition, the concentration of alginic acid in the obtained culture broth was measured to be 26.1 mg/L in terms of sodium alginate. From the results, the production volume of alginic acid per 1 kg of the dried alga was 31 g.

From the results of the above-described Examples and Reference Examples, it is clarified that a yield of alginic acid per a volume of a culture broth can be remarkably increased by heterotrophically cultivating an alga which belongs to the genus Parachlorella and has ability to produce alginic acid in comparison with the case that the alga is autotrophically cultivated. In addition, it is clarified that a yield of alginic acid relative to a weight of dried alga produced by cultivation can be also increased.

## Claims

1. A method for cultivating an alga,
comprising the step of cultivating the alga heterotrophically,
wherein the alga belongs to the genus Parachlorella and has ability to produce alginic acid.

2. The method according to claim 1, wherein the alga belonging to the genus Parachlorella is Parachlorella sp. binos FERM BP-10969.

3. The method according to claim 1 or 2, wherein the alga is cultivated under dark condition of less than 700 Lux on average illuminance.

4. A method according to any one of claims 1 to 3, wherein the alga is cultivated in a liquid culture medium, and a total amount of an organic compound to be used in the liquid culture medium is adjusted to not less than 2.5 g in terms of carbon atom weight relative to 1 L of the liquid culture medium at the start of the cultivation.

5. A method for producing an alginic acid-containing composition,
comprising the step of obtaining the alginic acid-containing composition from a culture obtained by the method according to any one of claims 1 to 4.

6. A dried alga, wherein
the dried alga is a dried product of the alginic acid-containing composition obtained by the method according to claim 5, and
a content of alginic acid per 1 kg is not less than 40 g.

7. A reduction agent of sludge, comprising the alginic acid-containing composition obtained by the method according to claim 5.

8. A harmful substance adsorption agent, comprising the alginic acid-containing composition obtained by the method according to claim 5.

9. A feed, comprising the alginic acid-containing composition obtained by the method according to claim 5.

10. A food, comprising the alginic acid-containing composition obtained by the method according to claim 5.

11. A medical drug, comprising the alginic acid-containing composition obtained by the method according to claim 5.
